Europäisches Patentamt

European Patent Office

Office européen des brevets

(11) Publication number: **0 000 634**
**A1**

# EUROPEAN PATENT APPLICATION

(21) Application number: **78300141.5**

(51) Int. Cl.²: **C 07 C 143/60**

(22) Date of filing: **12.07.78**

(30) Priority: **28.07.77 GB 31723/77**

(43) Date of publication of application:
**07.02.79 Bulletin 79/3**

(84) Designated contracting states:
**BE DE FR GB NL**

(71) Applicant: **The Clayton Aniline Company Limited**
**P.O. Box 2, Clayton**
**Manchester M11 4AP. (GB)**

(72) Inventor: **Hildreth, John David**
**Damson Cottage Andertons Lane Henbury**
**Macclesfield Cheshire. (GB)**

(72) Inventor: **Lynch, Joseph**
**7 Enfield Close Norden Rochdale**
**Lancashire. (GB)**

(74) Representative: **Sharman, Thomas CIBA-GEIGY (UK) Limited**
**Tenax Road Trafford Park**
**Manchester M17 1WT. (GB)**

(54) Process for the manufacture of naphthylamine sulphonic acids.

(57) A process for the manufacture of naphthylamine sulphonic acids comprises catalytically hydrogenating an alkali metal salt of the corresponding nitro-naphthalene sulphonic acid at a substantially neutral pH.

EP 0 000 634 A1

Croydon Printing Company Ltd

The present invention relates to the preparation of naphthylamine sulphonic acids.

Naphthylamine sulphonic acids are important starting materials for the production of dyestuffs by diazotisation followed by coupling with various coupling components. They are normally made by aqueous Béchamp reduction of the corresponding Nitro acid as its ammonium salt. While this process gives reasonably high yields of about 88%, the reduction period is prolonged. The reduction time is about 12 hours and this is followed by a further approximate 20 hours for ashing out of iron, filtration and washing of iron sludge. Also the process is carried out under slightly acidic conditions which causes corrosion problems, particularly when using stainless steel reactors.

Attempts at catalytic hydrogenation of the Nitro acids have been totally unsatisfactory, as large amounts of insoluble unknown impurity is formed. In addition, absorption of hydrogen quite frequently ceases very quickly, and catalyst poisoning is apparent.

We have now, surprisingly, found that the corresponding Nitro acid as its sodium salt can be catalytically hydrogenated easily and quickly in very high yields.

Accordingly, the present invention provides a process for the manufacture of naphthylamine sulphonic acids which comprises catalytically hydrogenating an alkali metal salt of the corresponding nitro-naphthalene sulphonic acid at a substantially neutral pH.

The process is suitable for the preparation of various naphthylamine sulphonic acids, such as 1-naphthylamine-sulphonic acid (Peri acid) 1-naphthylamine-5-sulphonic acid (Laurents acid) 1-naphthylamine-6-sulphonic acid (1,6-Cleves acid 1-naphthylamine-7-sulphonic acid (1,7-Cleves acid) and a mixture of the two isomers (mixed Cleves acid) or, preferably, 2-naphthylamine-4,8-disulphonic acid (C-acid).

The catalyst may be any that is known for carrying out catalytic hydrogenation reactions, such as nickel, ruthenium, rhodium, platinum and palladium. We prefer to use palladium on charcoal as the catalyst, in suitable catalytic amounts. For example, when using 5% palladium on charcoal, about 2% of catalyst relative to the Nitro acid is suitable. This is about 0.1% palladium based on the Nitro acid, although smaller amounts, e.g. 0.005% palladium based on the Nitro acid, can be used with more efficient agitation and mixing of the reactants. e.g. in a spray nozzle high circulation reactor such as a Buss loop reactor.

The alkali metal salt may be, for example, the sodium or potassium salt, but is preferably the sodium salt.

The pH of the reaction mixture should be substantially neutral and may range from 6.0 to 8.0, but is preferably from 7.0 to 7.5.

The reaction is carried out at elevated temperatures and elevated pressure. The reaction temperature may be from 60°C.

to 200°C., preferably from 90°C. to 120°C.    The pressure may be from 15 to 1400 psi, preferably from 70 to 420 psi.

The Nitro acid salt may be used at various concentrations, and may be used as a slurry in water or as a solution. At a temperature of 90 - 95°C. the salt is completely in solution at a concentration of 15% w/v.    At higher amounts of salt, some of it is out of solution.    In such cases it is preferred to dose the reaction mixture as the reaction proceeds with more of the salt, so enabling reactions at higher concentrations.    Such a process can be carried out, for example, in a Buss loop reactor.    The only upper limit to the concentration is that at the end of the reaction the resulting acid should be in solution, so that the catalyst can be removed by filtration.

The alkali metal salt of the Nitro acid may be obtained by reacting the ammonium salt which is normally used in the Béchamp reduction with caustic alkali, e.g. caustic soda liquor to pH 10.5.    The solution is then boiled to liberate ammonia after which the pH is re-adjusted to neutral, preferably at 90 - 95°C., prior to catalytic hydrogenation.    This may be carried out by adding a mineral acid such as hydrochloric acid.

To carry out the catalytic hydrogenation the following procedure is preferred.    The catalyst is added to the Nitro acid salt solution or slurry, the mixture is stirred and introduced into a pressure vessel. The vessel is then purged either with hydrogen or an inert gas such as nitrogen. With good agitation, hydrogen is then introduced under pressure, e.g. 70 p.s.i., and the mixture is heated to about 100°C. These conditions should be maintained until hydrogen is no longer taken up. This normally takes about 1 - 1$\frac{1}{4}$ hours, although the time may vary depending on the reaction temperature, pressure, amount of catalyst and efficiency of stirring, as is well known in catalytic reduction processes.

When no more hydrogen is taken up the reaction conditions are preferably maintained for a short period to ensure that the reduction is complete. This additional time may be up to about 30 minutes, but normally 15 minutes is sufficient.

The pressure is then released and the hot reaction solution filtered to remove the catalyst, and leave the desired acid in solution.

The acid may be recovered by precipitating from solution by acidifying the solution to pH 1 or below with a mineral acid such as hydrochloric acid, adding salt and cooling, followed by filtration. The amount of salt added may be from 10 % to 30 % w/v, but is preferably about 20% w/v. The mixture is preferably cooled to ambient temperature of, say, 15 - 25°C., to give a yield of acid of up to about 95% of theory. This yield can be increased even more by cooling to a lower temperature, for example to 5°C.

The invention is illustrated by the following Examples, in which parts by weight bear the same relationship to parts by volume as do kilograms to litres.

Example 1

A.    Preparation of the sodium salt of
      2-nitro-naphthalene-4,8-disulphonic acid

To 300 parts by volume of hot water were added, with stirring, 75.5 parts by weight of the ammonium salt of 2-nitro-naphthalene-4,8-disulphonic acid.    The mixture was heated to 90°C. to give complete solution and then 33 parts by weight of caustic soda liquor (s.g. 1.50) were added to give pH 10.5.    Water was added to bring the total volume up to 500 parts, and the solution was then boiled for $1\frac{1}{2}$ hours to remove ammonia.    The pH was then adjusted to 7.0 - 7.5 at 90 - 95°C. by adding 5.7 parts by weight of hydrochloric acid (s.g. 1.14).

B.    Preparation of 2-naphthylamine-4,8-disulphonic acid

To 500 parts by volume of the solution of the sodium salt prepared above were added 1.51 parts by weight of catalyst, comprising 5% palladium on charcoal, and the mixture was stirred for 10 minutes.    The mixture was then transferred into a pressure vessel which was closed and purged twice with hydrogen.    With agitation, hydrogen was introduced at 70 psi, and the mixture was heated to 100°C. After 1¼ hours the consumption of hydrogen ceased. The reaction conditions were then maintained for a further 15 minutes to ensure that the reduction was complete.

The pressure was then released and the catalyst filtered off, and washed with water.

40 Parts by weight of hydrochloric acid (s.g. 1.14) were added to the solution to reduce the pH to below 1. There was then added 109 parts by weight sodium chloride, the mixture was cooled to 20°C., and the product filtered off. The yield of product was 85 parts by weight, (92.5% theory).

Examples 2 - 5

The procedure described in Example 1B was repeated, using various concentrations, pH values and reaction times. The conditions and yields of product are set out in the Table below.

## TABLE

| Example No. | Reaction Conc. (w/v) Nitro C-acid | Catalyst (% Pd. on nitro) | pH of Reduction | Reduction Time | Isolation Conditions C-acid | Yield (% theory) |
|---|---|---|---|---|---|---|
| 2 | Approx. 16 | 0.1 | 7.5 | $1\frac{1}{4}$ h | pH 1.0 20% w/v NaCl 5°C. | 95.3 |
| 3 | 18 (ca. 10% nitro out of soln.) | 0.1 | 7.5 | 1 h | pH 1.0 20% w/v NaCl 20°C. | 93.8 |
| 4 | 15 | 0.1 | 7.3 | $1\frac{1}{4}$ h | pH 1.0 20% w/v NaCl 20°C. | 91.6 |
| 5 | 15 | 0.1 | 7.5 | 1 h 10 min. | pH 1.0 20% w/v NaCl 20°C. | 91.6 |

## Example 6

To a shaking autoclave was charged 500 parts by
volume of mixed 1,5- and 1,8-nitronaphthalene sulphonic
acids at 15% w/v concentration, and 3.75 parts by weight
of catalyst comprising 5% palladium on charcoal as a 50%
paste.   The reactor was then closed and purged twice with
hydrogen.   With agitation, hydrogen was then
introduced at 95° to 100°C. and 140 p.s.i. pressure, and
these conditions were maintained for 1 hour when consumption
of hydrogen ceased.   The reaction conditions were
maintained for a further 15 minutes to ensure that the
reduction was complete.   The pressure was then released
and the catalyst filtered off.

The mixed aminonaphthalene sulphonic acids were
separated as follows:

The solution containing the mixed isomers was cooled
to 0°C. and the pH reduced to 4.5 by adding hydrochloric
acid   After cooling to 50°C. at pH 4.5, the precipitated
1-aminonaphthalene-8-sulphonic acid (peri acid) was
recovered by filtration and washed with water.

The remaining mother liquors were then adjusted to
pH 1.5 - 2.0 by adding hydrochloric acid and cooled to
25°C.   1-aminonaphthalene-5-sulphonic acid (Laurents acid)
precipitated an was filtered off and washed with water.

What we claim is:

1.   A process for the manufacture of naphthylamine sulphonic acids characterised in that an alkali metal salt of the corresponding nitro-naphthalene sulphonic acid is catalytically hydrogenated at a substantially neutral pH.

2.   A process as claimed in claim 1 characterised in that the catalyst is nickel, ruthenium, rhodium, platinum or palladium.

3.   A process as claimed in claim 1 or 2 characterised in that the catalyst is palladium on charcoal.

4.   A process as claimed in any preceding claim characterised in that it is carried out at a temperature of from 60°C. to 200°C. and at a pressure of from 15 to 1400 psi.

5.   A process as claimed in claim 4 characterised in that it is carried out at a temperature of from 90°C. to 120°C. and at a pressure of 70 to 420 psi.

6.   A process as claimed in any preceding claim characterised in that the concentration of salt used is such that the resulting acid is in solution.

C000634

7. A process as claimed in any preceding claim characterised in that it is carried out in a loop reactor.

8. A process as claimed in any preceding claim characterised in that the resulting acid is recovered by lowering the pH to 1 or below with a mineral acid, adding salt, cooling and filtering.

9. A process as claimed in any preceding claim characterised in that the product is

1 - naphthylamine - 8 - sulphonic acid

1 - naphthylamine - 5 - sulphonic acid

1 - naphthylamine - 6 - sulphonic acid, and/or

1 - naphthylamine - 7 - sulphonic acid, or

2 - naphthylamine - 4,8 - disulphonic acid

10. A process as claimed in any preceding claim characterised in that the alkali metal salt is the sodium or potassium salt.

T. Sharman
Agent for the Applicants

## DOCUMENTS CONSIDERED TO BE RELEVANT

| Category | Citation of document with indication, where appropriate, of relevant passages | Relevant to claim |
|---|---|---|
| X | DE – B – 1 233 408 (BASF)<br>* Claim 1; column 2, lines 28 to 45 *<br>—— | 1,10 |
| X | US – A – 2 784 220 (L.SPIEGLER)<br>* Claim 1; example 1 *<br>—— | 1-3, 10 |
| X | FR – A – 2 213 936 (BASF)<br>* Claims 1,6,7,9,10 *<br>—— | 1,2,10 |
| X | JP – A – 50 59 349 (TAOKA DYESTUFFS)<br>* Pages 2-3 *<br>—————— | 1,2,4, 5,9,10 |

**CLASSIFICATION OF THE APPLICATION (Int. Cl.²)**

C 07 C 143/60

**TECHNICAL FIELDS SEARCHED (Int.Cl.³)**

C 07 C 143/60

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 25-10-1978 | MOREAU |

EPO Form 1503.1  06.78